# FASCICULE DE BREVET EUROPEEN

(11) **EP 2 790 642 B1**
(45) Date de publication et mention de la délivrance du brevet: **07.03.2018**
(21) Numéro de dépôt: 12810384.3
(22) Date de dépôt: 11.12.2012
(51) Int. Cl.: A61K 8/06, A61Q 17/04, A61K 8/37, A61K 8/04, A61K 8/40, A61K 8/49

(54) **KIT COSMETIQUE DE PROTECTION DE LA PEAU VIS-A-VIS DES RAYONS UV**
KOSMETISCHES KIT ZUM SCHUTZ DER HAUT GEGEN UV-STRAHLUNG
COSMETIC KIT FOR PROTECTING THE SKIN AGAINST UV RAYS

(30) Priorité: 14.12.2011 FR 1161600
(43) Date de publication de la demande: 22.10.2014
(73) Titulaire: LvmH Recherche, 45800 Saint-Jean De Braye (FR)
(72) Inventeur: ALARD, Valérie, 45000 Orléans (FR); BEAUFRERE-SERON, Béatrice, 45160 Olivet (FR); PERRIER, Eric, 38138 Les Cotes d'arey (FR); NOE, Brigitte, 45000 Orléans (FR)
(74) Mandataire: Mena, Sandra
(86) Numéro de dépôt international: PCT/FR2012/052870
(87) Numéro de publication internationale: WO 2013/088051

(56) Documents cités:
- FR-A1- 2 758 721
- DATABASE gnpd [Online] mintel; mars 2003 (2003-03), "Ultrasun skin care", XP002680683, Database accession no. 193523
- DATABASE gnpd [Online] mintel; juin 2011 (2011-06), "Sun Spray spf10 + after sun set", XP002680684, Database accession no. 1559246
- DATABASE gnpd [Online] mintel; juin 2007 (2007-06), "Sun safety kit", XP002680685, Database accession no. 717160
- DATABASE gnpd [Online] mintel; juin 2008 (2008-06), "Summer Skin Travel Essentials", XP002680686, Database accession no. 923822
- DATABASE gnpd [Online] mintel; janvier 2009 (2009-01), "Sun kit", XP002680687, Database accession no. 1029427
- DATABASE gnpd [Online] mintel; mai 2009 (2009-05), "Self-Tanning & Anti-Aging Facial Cream Set", XP002680688, Database accession no. 1083564
- DATABASE gnpd [Online] mintel; mai 2010 (2010-05), "UV Special Care Set", XP002680689, Database accession no. 1322879
- DATABASE GNPD MINTEL; juin 2007 (2007-06), "Bronze Have More Fun!", XP002697248, Database accession no. 719963

## Description

L'invention a pour objet un kit de protection de la peau contre les rayons ultraviolets (UV). Il est utilisé dans une méthode qui consiste à appliquer sur la peau avant ou pendant une exposition au soleil la première composition du kit, puis à réappliquer, pendant l'exposition au soleil la deuxième composition cosmétique du kit d'indice de protection solaire inférieur à celui de ladite première composition pour prolonger la protection de la peau.

### ETAT DE L'ART et BUT de L'INVENTION

Pour être efficacement protégé contre les effets néfastes des rayons ultraviolets (« rayons UV »), les professionnels de santé recommandent d'appliquer sur la peau, préalablement à toute exposition au soleil, une composition contenant des filtres UV organiques, notamment liposolubles, et minéraux dont le niveau de protection est élevé, généralement supérieur à un indice de 20.

Les filtres UV minéraux sont des filtres UV très efficaces contre les fortes expositions aux UV car ils réfléchissent et diffusent la lumière. Les filtres UV organiques liposolubles ont quant à eux une forte substantivité sur la peau.

Il est également conseillé de renouveler l'application de cette composition régulièrement pendant toute la durée de l'exposition au soleil. En effet, il a été constaté que le niveau de protection solaire conféré par une crème ou une lotion diminue au cours du temps après son application sur la peau, en raison notamment de la diminution de l'épaisseur du dépôt de produit provoqué par des frottements, un lavage à l'eau ou au savon, la transpiration, ou même le simple séchage du produit sur la peau. Il est en pratique recommandé de réitérer régulièrement l'application du produit, généralement au bout de deux heures environ, ou plus tôt si par exemple la zone de peau sur laquelle a été appliquée la composition a été lavée ou exposée à de l'eau de mer, ou encore si la personne a transpiré en faisant de l'exercice physique.

Les produits de protection solaire, qualifiés de produits à fort indice de protection solaire, qui sont utilisés pour se protéger en cas d'exposition intense au soleil ont généralement une texture assez épaisse et des propriétés sensorielles à l'application peu satisfaisantes, principalement causées par la présence en fortes teneurs de filtres UV minéraux ou de filtres UV organiques liposolubles.

Il existe donc un besoin d'améliorer le confort des produits à fort indice de protection solaire tout en maintenant un niveau de protection élevé.

Les inventeurs ont trouvé de façon surprenante que la prolongation et le maintien du niveau de protection vis-à-vis des ultraviolets, initialement conféré par un premier dépôt de produit solaire ne nécessitent pas obligatoirement de réappliquer sur la peau la même composition. Avec le kit de l'invention, il est proposé de maintenir et prolonger le niveau de protection du dépôt initial réalisé avant une exposition solaire, grâce à l'application après un certain temps, habituellement deux heures après application du premier dépôt, d'une deuxième composition dont l'indice de protection est inférieur à celui de la première composition appliquée. Cette nouvelle méthode permet avantageusement de ne pas réappliquer la première composition, mais d'utiliser un deuxième produit dont la texture est bien plus agréable à appliquer sur les zones de peau à protéger. Le kit de l'invention permet ainsi d'améliorer le confort d'utilisation d'un produit solaire à fort indice de protection sans diminuer le niveau de protection de la peau du consommateur.

Il a déjà été proposé de combiner l'application de deux produits différents pour protéger la peau des rayons UV.

Dans la demande FR 2 758 721 par exemple, on applique une composition ne contenant que des filtres UVB pendant les premiers jours d'exposition au soleil jusqu'à ce que la peau commence à bronzer. Dès l'apparition du bronzage, généralement après 3 à 6 jours d'exposition, on utilise un autre produit filtrant les UVA. Dans cette méthode de protection solaire, on ne superpose pas deux produits l'un sur l'autre dans la même journée. On ne répond donc pas au besoin exprimé plus haut.

Dans la demande FR 2 933 614, on cherche à réaliser un film de composition solaire sur la peau en faisant réagir de façon extemporanée deux produits l'un avec l'autre. Dans cette méthode de protection solaire, on ne superpose pas deux produits l'un sur l'autre de façon décalée dans le temps, mais on les mélange au moment de leur application sur la peau. Une réaction chimique se produit lorsque les deux produits sont mis en présence, et conduit à la formation d'une pellicule souple et protectrice à la surface de la peau.

### DESCRIPTION DE L'INVENTION

### Méthode

La présente invention concerne strictement un kit de protection solaire tel que revendiqué et non une méthode de protection de la peau. Dans le texte qui suit, il faut interpréter l'expression "méthode de l'invention" comme étant une "méthode d'utilisation du kit de l'invention tel que revendiqué". Une méthode de protection de la peau contre les rayons UV utilisant deux compositions cosmétiques de protection contre les rayons UV ayant des valeurs d'indice de protection solaire différentes, consiste:
- à appliquer sur la peau une première composition ayant un certain indice de protection solaire,
- à exposer la peau au soleil, puis
- à appliquer sur le dépôt résiduel de la première composition une seconde composition cosmétique ayant un indice de protection solaire inférieur à celui de la première composition.

La méthode de protection de la peau contre les rayons UV est de préférence mise en oeuvre lorsque la peau est exposée au soleil de façon intensive parce que l'ensoleillement est important ou plus important que la normale d'exposition de l'individu exposé, et/ou parce que la durée d'exposition est plus longue que la durée moyenne d'exposition que subit l'individu exposé.

La première composition peut être appliquée juste avant ou pendant l'exposition au soleil, tandis que la deuxième composition est appliquée pendant l'exposition au soleil pour prolonger les effets de protection conférés par la première composition.

Préalablement à la mise en oeuvre de la méthode de l'invention, on a avantageusement mesuré l'indice de protection solaire, et de préférence le facteur de protection solaire SPF, de chacune des deux compositions selon la même méthode de mesure de manière à pouvoir s'assurer qu'elles ont bien des valeurs d'indice de protection différentes.

### Définition et mesure de l'indice de protection solaire

Le niveau de protection solaire conféré par chaque composition ainsi que par la superposition des dépôts de chacune des compositions peut être mesuré par diverses méthodes connues de l'homme du métier réalisées *in vivo* ou *in vitro.*

L'indice de protection solaire d'une composition peut être mesuré *in vivo* selon la méthode de mesure du facteur de protection solaire (SPF Sun Protection Factor) publiée par le Colipa, le CTFA SA, le JCIA et le CTHA en mai 2006 (sur le site http://www.colipa.eu/publications-colipa-the-european-cosmetic-cosmetics-association; « International Sun Protection Factor Test Method - 2006 »). Selon cette méthode, le SPF d'une composition est défini comme le rapport entre le temps d'irradiation nécessaire pour atteindre le seuil érythématogène de la peau sur laquelle a été appliquée la composition, et le temps nécessaire pour atteindre le seuil érythématogène de la peau nue. La méthode publiée par le Colipa précise les conditions minimales à respecter pour que la mesure du SPF soit reproductible et significative. Les directives mentionnent notamment quelle quantité de composition doit être appliquée sur la peau, et quelle lampe d'irradiation doit être utilisée.

On entend par « facteur de protection solaire SPF » ou « SPF » au sens de l'invention, l'indice de protection solaire de la composition mesurée selon cette méthode. On retrouve la mention de la valeur de ce facteur de protection solaire SPF sur l'emballage des compositions cosmétiques de protection solaire, ce qui vise à informer le consommateur du niveau de protection dont il bénéficiera en appliquant ladite composition sur les zones de peau exposées.

Il existe également d'autres méthodes permettant de quantifier le niveau de protection conféré par un produit cosmétique contre les rayons UV, comme par exemple la méthode PPD (Persistent Pigment Darkening), qui mesure la couleur de la peau observée 2 à 4 heures après une exposition de la peau aux UV-A (longueurs d'onde comprises entre de 320 nm à 400 nm). Cette méthode est utilisée depuis 1996 par la Japanese Cosmetic Industry Association (JCIA) pour l'étiquetage UV-A des produits et par les laboratoires de tests en Europe et aux Etats-Unis (Japan Cosmetic Industry Association - Technical Bulletin - Measurement Standards for UVA protection efficacy - Issued November 21, 1995 and effective of January 1, 1996). Le facteur de protection UVAPPD (FP UVAPPD) correspond au rapport i) de la dose de rayonnement UV-A nécessaire pour que la peau recouverte de composition cosmétique atteigne le seuil de pigmentation (MPPDp) à ii) la dose de rayonnement UV-A nécessaire pour que la peau nue atteigne le seuil de pigmentation (MPPDnp).

La méthode de l'invention ne comprend elle-même aucune étape de mesure de l'indice de protection solaire.

Pour la méthode de l'invention, on mesure préalablement à sa mise en oeuvre, l'indice de protection solaire de chacune des deux compositions par la même méthode de mesure. On peut ainsi comparer le niveau de protection solaire conféré par l'une ou l'autre des compositions et établir un ordre d'application en fonction des indices mesurés.

Selon le même principe, on a utilisé dans les exemples illustrant l'invention, la même méthode de mesure à la fois pour déterminer l'indice de protection solaire de chaque composition, mais également pour déterminer le niveau de protection résultant de la superposition des deux compositions l'une sur l'autre, après mise en oeuvre de la méthode de l'invention.

Dans le cadre de l'invention, on préfère mesurer le niveau de protection solaire que fournissent la première composition et la deuxième composition, en utilisant la méthode préconisée par le Colipa en 2006. L'indice de protection solaire mesuré pour la première composition, pour la deuxième composition, ainsi que pour le niveau de protection fourni résultant de la superposition des deux compositions l'une sur l'autre, est le SPF.

Cependant, on peut également mesurer le niveau de protection solaire que fournissent la première composition, la deuxième composition, ainsi que la superposition des deux compositions l'une sur l'autre, en utilisant une variante de la méthode préconisée par le Colipa en 2006, la dose de produit appliqué sur la peau, étant inférieure ou égale à 1 mg/cm², par exemple égale à 0,8 mg/cm². Dans ce cas, on ne parle plus de « facteur de protection solaire » ou de « SPF » mais simplement d'« indice de protection solaire ».

Dans la méthode de l'invention, la deuxième composition est de préférence appliquée sur la première composition dès que celle-ci a séché. On entend par séchage, l'évaporation au moins partielle d'au moins un ingrédient de ladite première composition, une fois que celle-ci a été appliquée sur la peau. L'application de la deuxième composition est réalisée dans la même journée que l'application de la première composition. Aussi, lorsque la première composition contient de l'eau, l'eau s'évapore au moins partiellement après l'application de la composition sur la peau, et la deuxième composition est de préférence appliquée sur le dépôt résiduel de la première composition qui a au moins partiellement séché.

La méthode de l'invention permet avantageusement de prolonger le niveau de protection solaire au cours du temps grâce à l'application - au bout d'un certain temps après l'application d'une première composition - d'un deuxième produit dont l'indice de protection solaire est plus faible.

La méthode de l'invention permet d'obtenir de façon inattendue un effet synergique, puisque la superposition de la deuxième composition sur le film résiduel de la première composition permet de retrouver le niveau de protection initial.

La protection conférée vis-à-vis des UV par l'application successive des deux compositions selon la méthode de l'invention, mesurée par exemple par le SPF, est supérieure à la somme de la protection conférée par la deuxième composition et de la protection conférée par le film résiduel de la première composition. Aussi, la superposition des deux compositions n'entraîne pas une simple addition des protections générées par chaque composition prise individuellement, mais produit bien un effet de synergie qui n'était pas prévisible par l'homme du métier.

Dans la méthode de l'invention, les deux compositions ne sont pas destinées à être mélangées à la main préalablement à leur application, ou après leur application sur la peau. On cherche au contraire à superposer les dépôts de chaque composition sur la peau. Aussi, la deuxième composition est avantageusement appliquée à la surface du dépôt de la première composition sur la peau, et la personne qui applique le produit ne cherche pas - au moment de l'application de la deuxième composition sur la première - à détruire l'intégrité de la première couche. En particulier, les deux compositions ne contiennent pas des ingrédients susceptibles de réagir chimiquement entre eux par la création de liaisons covalentes.

La première composition est de préférence appliquée en une quantité suffisante pour recouvrir la surface de la peau à protéger des effets des ultraviolets. La deuxième composition est de préférence appliquée en une quantité suffisante pour recouvrir le dépôt résiduel de la première composition.

Le séchage de la première composition peut être effectué en exposant la peau au soleil, pendant une durée d'au moins 10 minutes. L'application de la deuxième composition est de préférence effectuée au bout d'une durée d'exposition au soleil n'excédant pas 2 heures.

A titre indicatif, il est habituellement recommandé par les experts de renouveler l'application d'un produit solaire, au plus deux heures après la première application.

La méthode de l'invention ne vise pas à allonger le délai de ré-application d'un produit solaire, mais à prolonger la protection en appliquant à distance de la première application, une deuxième composition dont l'indice de protection est plus faible. Un délai de deux heures entre l'application initiale de la première composition et l'application de la deuxième composition est ainsi tout à fait compatible avec la méthode de l'invention.

L'indice de protection solaire mesuré après le dépôt de la seconde composition superposé au dépôt résiduel de la première composition est avantageusement au moins égal à l'indice de protection solaire de la première composition.

Selon la méthode de l'invention, au-delà de la première ré-application de produit, correspondant à la première application de la deuxième composition sur le dépôt résiduel de la première composition, il est tout à fait possible de réappliquer à nouveau la deuxième composition sur la zone de peau à protéger de façon à renouveler le film de protection vis-à-vis des rayons ultraviolets.

### Première composition

Un dépôt de la première composition présente avantageusement un indice de protection solaire supérieur à celui de la deuxième composition, à quantité de compositions égales.

La première composition présente un indice de protection solaire SPF, supérieur ou égal à 30, de préférence supérieur ou égal à 40, de préférence encore supérieur ou égal à 50. En outre la première composition se présente sous la forme d'une émulsion contenant au moins un filtre UV liposoluble. La première composition peut présenter elle-même un SPF de 30.

### Formule de référence A de SPF 30

| **DENOMINATION INCI** | **% Massique** |
|---|---|
| EAU | qsp 100 |
| BUTYLENE GLYCOL DICAPRYLATE / DICAPRATE | 9,8 |
| ETHYLHEXYL METHOXYCINNAMATE | 7,5 |
| DICAPRYLYL CARBONATE | 4,0 |
| BUTYLENE GLYCOL | 3,8 |
| METHYLENE BIS-BENZOTRIAZOLYL TETRAMETHYLBUTYLPHENOL | 3,5 |
| BIS-ETHYLHEXYLOXYPHENOL METHOXYPHENYL TRIAZINE | 3,0 |
| BEHENYL ALCOHOL | 2,2 |
| CAPRYLYL METHICONE | 2,0 |
| VP/EICOSENE COPOLYMER | 2,0 |
| GLYCEROL | 2,0 |
| POTASSIUM CETYL PHOSPHATE | 2,0 |
| DIMETHICONE | 2,0 |
| CETEARYL ALCOHOL | 1,2 |
| PHENYLBENZIMIDAZOLE SULFONIC ACID | 1,0 |
| TROMETHAMINE | 0,9 |
| PHENOXYETHANOL | 0,9 |
| HUILE COCOS NUCIFERA (COCONUT) | 0,9 |
| PHENYL TRIMETHICONE | 0,8 |
| CAPRYLYL GLYCOL | 0,6 |
| DECYL GLUCOSIDE | 0,5 |
| GOMME XANTHANE | 0,3 |
| CETEARYL GLUCOSIDE | 0,3 |
| SILICE | 0,2 |
| TETRASODIUM EDTA | 0,2 |
| TOCOPHERYL ACETATE | 0,2 |
| CARBOMER | 0,1 |
| ACTIFS COSMETIQUES | 0,2 |

### Filtres UV

La première composition contient au moins un filtre UV qui peut être choisi parmi les filtres UV organiques hydrophiles, les filtres UV organiques liposolubles et les filtres UV minéraux. Elle doit comprendre au moins un filtre UV liposoluble. Par "filtre UV organique hydrophile", on entend tout composé organique absorbant un rayonnement ultra-violet (UV) dans la gamme de longueurs d'onde allant de 280 nm à 400 nm qui peut être dissous dans la phase aqueuse de la composition, ou qui peut y être dispersé sous forme colloïdale ou sous forme micellaire.

Parmi les filtres UV hydrophiles, on peut utiliser les filtres UV suivants désignés ci-dessous par leur nom INCI ou leur nom chimique:
- l'acide téréphthalylidène dicamphosulfonique (Nom INCI : Terephthalylidène Dicamphre Acide Sulfonic Acid) commercialisé sous le nom MEXORYL® SX par CHIMEX,
- les dérivés du bis-benzoazolyle tels que décrits dans les brevets EP 669 323 et US 2,463,264 et plus particulièrement le composé Disodium Phenyl Dibenzimidazole Tetra-sulfonate vendu sous le nom commercial commercial NEO HELIOPAN® AP par Haarmann et Reimer,
- l'acide p-aminobenzoïque (Nom INCI : PABA) et ses dérivés, tels que le 1-(4-aminobenzoate)-1,2,3-propanetriol (nom INCI : Glyceryl PABA) et le PEG-25 PABA vendu sous le nom UVINUL® P25 par BASF,
- l'acide 2-phénylbenzimidazole-5-sulfonique (nom INCI : Phenylbenzimidazole Sulfonic Acid) vendu notamment sous le nom commercial EUSOLEX® 232 par MERCK,
- le salicylate de triéthanolamine,
- le 3-(4'-sulfobenzylidène) camphre (nom INCI : Benzylidene camphor sulfonic acid) commercialisé sous le nom MEXORYL® SL par CHIMEX,
- le Méthylène bis-Benzotriazolyl Tétraméthylbutylphénol (Dénomination USAN : BISOCTRIZOLE) vendu sous la référence Tinosorb® M, ou MIXXIM® BB/100 par FAIRMOUNT CHEMICAL;
- le 3-(4'-triméthylammonium benzylidène)-1-bornan-2-one méthyl sulfate (Nom INCI : Camphor Benzalkonium Methosulfate) commercialisé sous le nom "MEXORYL SO" par CHIMEX,
- la Benzophénone-4 vendu sous le nom commercial UVINUL® MS40.

On peut également utiliser comme filtre organique UV hydrophile, des molécules organiques filtrant les UV qui sont de nature lipophile (dissoutes ou dispersées dans un liquide non aqueux) qui ont été rendues hydrophiles par adsorption sur un support hydrophile de faible granulométrie, comme des particules de polymère. On pourra citer par exemple la bis-éthylhexyloxyphénol méthoxyphényl triazine, qui est un filtre UV lipophile adsorbé sur des particules de polyméthacrylate de méthyle (PMMA). Le filtre UV organique hydrophile peut donc être une molécule organique lipophile filtrant les UV, adsorbée ou absorbée sur un support hydrophile, lequel peut ne pas filtrer les UV, tel qu'un polymère organique.

On préfère utiliser un filtre UV hydrophile choisi parmi le bis-éthylhexyloxyphénol méthoxyphényl triazine, la benzophénone-4, l'acide 2-phénylbenzimidazole-*5*-sulfonique ou un de leurs mélanges.

Selon un mode de réalisation, elle contient des filtres UV organiques liposolubles et des filtres UV minéraux.

Par "filtre UV organique liposoluble", on entend tout composé organique absorbant un rayonnement UV dans la gamme de longueurs d'onde allant de 280 nm à 400 nm qui peut être dissous à l'état moléculaire dans une huile, ou être dispersé dans une huile sous forme colloïdale ou sous forme micellaire.

Les filtres UV organiques liposolubles peuvent notamment être choisis parmi différentes familles de composés chimiques. On peut notamment citer les dérivés de l'acide para-aminobenzoïque, les dérivés salicyliques, les dérivés cinnamiques, les aminobenzophénones, les dérivés anthraniliques, les dérivés du dibenzoylméthane, les dérivés de [beta],[beta]'-diphénylacrylate, les dérivés du benzylidène camphre, les dérivés du phényl benzotriazole, les dérivés triazine, les bis-résorcinyl triazines, les dérivés d'imidazolines, les dérivés du benzalmalonate, les dérivés de 4,4-diarylbutadiène, les dérivés de benzoxazole, les mérocyanines et leurs mélanges.

Des exemples de dérivés de l'acide para-aminobenzoïque sont l'Ethyl PABA, l'Ethyl Dihydroxypropyl PABA et l'Ethylhexyl Diméthyl PABA.

Des dérivés salicyliques sont notamment l'Homosalate vendu notamment sous le nom" Eusolex HMS®" par Rona/EM Industries ; l'Ethylhexyl Salicylate vendu notamment sous le nom" NEO HELIOPAN OS®" par SYMRISE ; le Dipropylèneglycol Salicylate vendu notamment sous le nom" DIPSAL® " par SCHER.

Parmi les dérivés du cinnamate, on peut citer notamment de manière non limitative: l'éthyl-2-hexyl-p-methoxycinnamate, l'isopropyl-p-methoxycinnamate, Isoamyl Methoxycinnamate, le Cinoxate (2-éthoxyéthyl-p-methoxycinnamate), Diéthanolamine Methoxycinnamate, Glyceryl Ethyl-2-hexanoate Di-p-methoxycinnamate, [4-bis(trimethylsiloxy)methylsilyl-3-methylbutyl]-3,4,5,-trimethoxycinnamate.

Parmi les dérivés du cinnamate mentionnés ci-dessus, on utilisera tout particulièrement l'éthyl-2-hexyl-p-methoxycinnamate encore appelé Ethylhexyl Methoxycinnamate ou Octyl Methoxycinnamate (de dénomination USAN : Octinoxate) proposé à la vente sous les dénominations commerciales PARSOL MCX de la Société DSM NUTRITIONAL PRODUCTS, UVINUL MC 80 de la société BASF.

Parmi les dérivés de benzophénone, on citera la Benzophénone-1 vendu sous le nom commercial UVINUL® 400; la Benzophénone-2 vendu sous le nom commercial UVINUL D50; la Benzophénone-3 ou Oxybenzone vendu sous le nom commercial UVINUL® M40; la Benzophénone-6 vendu sous le nom commercial HELISORB 11; et la Benzophénone-8 vendu sous le nom commercial SPECTRASORB® UV-24.

Une aminobenzophénone est par exemple le 2-(4-diéthylamino-2-hydroxybenzoyl)-benzoate de n-hexyle vendu notamment sous le nom commercial " UVINUL® A +" par BASF.

Parmi les dérivés anthraniliques, on citera le menthylanthranilate vendu notamment sous la référence NEO HELIOPAN® MA par SYMRISE.

Parmi les filtres UV dérivés du dibenzoylméthane, on peut notamment citer, de manière non limitative: le 2-méthyldibenzoylméthane, le 4-méthyldibenzoylméthane, le 4-isopropyldibenzoylméthane, le 4-tert-butyldibenzoylméthane, le 2,4-diméthyldibenzoylméthane, le 2,5-diméthyldibenzoylméthane, le 4,4'-diisopropyldibenzoylméthane, le 4,4'-diméthoxydibenzoylméthane, le 4-tert-butyl-4'-méthoxydibenzoylméthane, le 2-méthyl-5-isopropyl-4'-méthoxydibenzoylméthane, le 2-méthyl-5-tert-butyl-4'-méthoxydibenzoylméthane, le 2,4-diméthyl-4'-méthoxydibenzoylméthane, le 2,6-diméthyl-4-tert-butyl-4'-méthoxydibenzoylméthane.

Parmi les dérivés du dibenzoylméthane mentionnés ci-dessus, on utilisera tout particulièrement le 4-(ter-butyl)-4'-méthoxy dibenzoylméthane encore appelé Butyl Methoxy Dibenzoylmethane (en abrégé BMDBM, de dénomination INCI 1-[4-(1,1-dimethylethyl)-phenyl]-3-(4-methoxyphenyl)-1,3-propanedione et de dénomination USAN Azobenzone) proposé à la vente sous les dénominations commerciales de PARSOL® 1789 de la Société DSM NUTRITIONAL PRODUCTS, ou EUSOLEX® 9020 de la société MERCK.

Deux dérivés du [beta],[beta]'-diphénylacrylate sont l'Octocrylène, vendu notamment sous le nom commercial " UVINUL® N539 " par BASF ; et l'Etocrylène, vendu notamment sous le nom commercial " UVINUL® N35 " par BASF.

Des exemples de dérivés du benzylidène camphre sont le 3-Benzylidène camphre ; le Méthylbenzylidène camphre vendu notamment sous le nom " EUSOLEX® 6300 " par MERCK ; et le Polyacrylamidométhyle Benzylidène Camphre.

On peut citer comme dérivé du phényl benzotriazole le Drométrizole Trisiloxane vendu notamment sous le nom "Silatrizole®" par RHODIA CHIMIE.

Parmi les dérivés de triazine ; on peut citer l'Ethylhexyl triazone vendu notamment sous le nom commercial " UVINUL® T150 " par BASF ; le Diéthylhexyl Butamido Triazone vendu notamment sous le nom commercial " UVASORB® HEB " par SIGMA 3V ; le 2,4,6-tris(4'-amino benzalmalonate de dinéopentyle)-s-triazine ; le 2,4,6-tris(4'-amino benzalmalonate de diisobutyle)-s- triazine ; le 2,4-bis(4'-amino benzalmalonate de dinéopentyle)-6-(4'-aminobenzoate de n-butyle)-s-triazine ; le Bis- EthylhexyloxyphenolMethoxyphenyl-Triazine et le 2,4-bis(4'-amino benzoate de n-butyle)-6-(aminopropyltrisiloxane)-s-triazine.

Un dérivé bis-résorcinyl triazines est le Bis-Ethylhexyloxyphénol Méthoxyphenyl Triazine vendu notamment sous le nom commercial " TINOSORB® S" par CIBA GEIGY.

Un dérivé d'imidazoline est l'Ethylhexyl Diméthoxybenzylidène Dioxoimidazoline Propionate.

Des dérivés du benzalmalonate sont les polyorganosiloxanes à fonction benzalmalonate tels que le Polysilicone-15 vendu notamment sous la dénomination commerciale " PARSOL® SLX " par DSM Nutritional Products, Inc. ; et le Di-néopentyl 4'-méthoxybenzalmalonate.

Un dérivé de benzoxazole est le 2,4-bis-[5-1(diméthylpropyl)benzoxazol-2-yl-(4-phenyl)-imino]-6-(2-ethylhexyl)-imino-1,3,5-triazine vendu notamment sous le nom d'UVASORB® K2A par Sigma 3V.

Un dérivé de mérocyanine est l'Octyl-5-N,N-diéthylamino-2-phénysulfonyl-2,4-pentadiénoate.

Un exemple de dérivé de 4,4-diarylbutadiène est le 1,1-dicarboxy (2,2'-diméthyl-propyl)-4,4-diphénylbutadiène.

Dans le cadre de la présente invention, on choisit de préférence un filtre UV organique liposoluble parmi les filtres UV suivants et leurs mélanges: l'Ethylhexylsalicylate ; l'Octocrylène ; l'Ethylhexyl triazone ; l'Ethylhéxyl Méthoxycinnamate ; le Butyl Méthoxydibenzoylméthane ; la Bis-Ethylhexyloxyphénol Méthoxyphenyl Triazine ; ou l'Oxybenzone.

La première composition peut également contenir au moins un filtre UV minéral.

Les filtres UV minéraux peuvent être choisis parmi des pigments d'oxydes métalliques de taille moyenne des particules généralement comprises entre 5 nm et 100 nm, de préférence entre 10 nm et 50 nm comme par exemple des pigments d'oxyde de titane (amorphe ou cristallisé sous forme rutile et/ou anatase), de fer, de zinc, de zirconium ou de cérium.

Les pigments peuvent être traités en surface ou non.

Les pigments traités en surface sont des pigments qui ont subi un ou plusieurs traitements de surface de nature chimique, électronique, mécanochimique et/ou mécanique avec des composés tels que décrits par exemple dans Cosmetics & Toiletries, Février 1990, Vol. 105, p. 53-64 , tels que des aminoacides, de la cire d'abeille, des acides gras, des alcools gras, des tensio-actifs anioniques, des lécithines, des sels de sodium, potassium, zinc, fer ou aluminium d'acides gras, des alcoxydes métalliques (de titane ou d'aluminium), du polyéthylène, des silicones, des protéines (collagène, élastine), des alcanolamines, des oxydes de silicium, des oxydes métalliques ou de l'hexamétaphosphate de sodium.

Les silicones utilisées pour le traitement des pigments sont par exemple choisies dans le groupe contenant les alkyl silanes, les polydialkylsiloxanes, et les polyalkylhydrogénosiloxanes.

Bien entendu, les pigments d'oxydes métalliques avant leur traitement par des silicones, peuvent avoir été traités par d'autres agents de surface, en particulier par de l'oxyde de cérium, de l'alumine, de la silice, des composés de l'aluminium, des composés du silicium, ou leurs mélanges.

Dans le cadre de l'invention on préfère utiliser l'oxyde de titane amorphe ou cristallisé sous forme rutile et/ou anatase comme filtre UV minéral.

La teneur en filtre UV dans la première composition est par exemple comprise entre 0,5 à 40 % en poids, particulièrement de 5 à 30 % en poids, plus particulièrement de 10 à 20 % en poids, par rapport au poids total de la composition.

La teneur en filtre UV liposoluble dans la première composition est par exemple comprise entre 5 à 15 % en poids, particulièrement de 10 à 12 % en poids, par rapport au poids total de la composition.

La teneur en filtre UV hydrophile dans la première composition est par exemple comprise entre 1 à 10 % en poids, particulièrement de 4 à 6 % en poids, par rapport au poids total de la composition.

La première composition contient de préférence de 0 à 5% en poids de filtres UV minéraux, et de manière encore préférée, elle n'en contient pas.

La première composition contient de préférence au moins un filtre filtrant les UV_{A} et au moins un filtre filtrant les UV_{B}. On entend par UV_{A} les longueurs d'onde allant de 315 à 400 nm, et par UV_{B} les longueurs d'ondes allant de 280 à 315 nm. Un filtre organique actif dans l'UV_{A} est avantageusement choisi parmi:
- les dérivés du dibenzoylméthane,
- le menthylanthranilate vendu notamment sous la référence NEO HELIOPAN® MA par SYMRISE, et
- leurs mélanges.

La première composition comprend de préférence les filtres UV suivants, pris seuls ou en mélange : l'éthyl-2-hexyl-p-methoxycinnamate, le Méthylène bis-Benzotriazolyl Tétramethylbutylphénol, la Bis- EthylhexyloxyphénolMéthoxyphényl-Triazine, l'acide 2-phénylbenzimidazole-5-sulfonique.

### Huile polaire non volatile

La première composition contient de préférence une huile polaire non volatile qui permet la solubilisation du filtre UV contenu dans la deuxième composition.

Par huile polaire non volatile, on entend, au sens de l'invention, un corps gras, non soluble dans l'eau, liquide à 25°C et 0,1 MPa, et non volatile ayant une pression de vapeur, à 25°C et 0,1 MPa, non nulle inférieure à 2,6 Pa, de préférence inférieure à 0,13 Pa, qui contient au moins un, de préférence au moins deux atomes d'oxygène ou des doubles liaisons conjuguées.

L'huile est de préférence choisie parmi les mono- et di-esters aliphatiques, les esters aromatiques non hydroxylés, les carbonates aliphatiques et les silicones phénylés.

Comme huiles polaires, on peut citer par exemple
- les mono-et di-esters aliphatiques, notamment i) les mono-esters d'un acide carboxylique aliphatique linéaire ou ramifié, saturé ou insaturé, de préférence saturé, comprenant de 8 à 20 atomes de carbone, et d'un mono-alcool aliphatique comprenant de 3 à 20 atomes de carbone, ii) les di-esters aliphatiques d'un di-acide aliphatique carboxylique comprenant de 4 à 10 atomes de carbone et d'un mono-alcool,
- les mono-esters de l'acide benzoïque et d'un alcool aliphatique comprenant de 8 à 20 atomes de carbone, le benzoate d'éthyle-2-hexyle, le benzoate d'octyl-2-dodécyle, le benzoate d'isostéaryle, le C12-C15 alkylbenzoate,
- les tri et tétra-esters tels que les esters du pentaérytritol, notamment le tétraisoséarate de pentaerythrityle, les esters du triméthylolpropane, notamment le triisoséarate de triméthylolpropane, les esters de l'acide citrique, notamment le citrate tridécyle, et le triméllitate de tridécyle,
- les di-alkyl-carbonates dont les groupes alkyles contiennent de 8 à 18 atomes de cabone tels que le dicaprylyl-carbonate, le di-(éthyl-2-hexyl)-carbonate,
- les mono ou di-esters aliphatiques hydroxylés tels que i) les esters d'un mono ou di-acide carboxylique aliphatique hydroxylé comprenant de 3 à 20 atomes de carbone, et d'un mono-alcool aliphatique aliphatique comprenant de 6 à 20 atomes de carbone, par exemple le lactate d'isostéaryle, l'hydroxystéarate d'octyle, l'hydroxystéarate d'octyldodécyle, le lactate de cétyle, le lactate de myristyle, le diisostéaryl-malate, ou ii) les mono et di-esters aliphatiques de polyol, en particulier de diols et de triols, tels que les esters d'un mono acide carboxylique aliphatique comprenant de 3 à 20 atomes de carbone, et d'un diol ou d'un triol aliphatique comprenant de 3 à 20 atomes de carbone,
- les mono- et di-esters hydroxylés aromatiques d'un acide carboxylique aromatique hydroxylé et d'un mono-alcool aliphatique comprenant au moins 10 atomes de carbone,
- les alcools aliphatiques saturés ou insaturés ayant de 8 à 26 atomes de carbone, comme l'octyldodécanol, l'octyldécanol, le 2-butyloctanol, le 2-hexyldécanol, le 2-undécylpentadécanol,
- les acides mono-carboxyliques aliphatiques saturés ou insaturés ayant de 7 à 29 atomes de carbone tels que l'acide oléique, l'acide linoléique, l'acide linolénique ou l'acide isostéarique,
- les huiles de silicone comportant au moins un groupement alcoxy ou phényle, pendant ou en bout de chaîne siliconée, ayant de 2 à 24 atomes de carbone, notamment la phényltriméthicone, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes;
- les glycols,
- les éthers aliphatiques comprenant plus de 10 atomes de carbone,
- les triglycérides, par exemple les huiles de tournesol, de maïs, de soja, de courge, de pépins de raisin, de sésame, de noisette, d'abricot, de macadamia, d'arara, de tournesol, de ricin, d'avocat, de jojoba, de beurre de karité, les triglycérides des acides caprylique/caprique, le triheptanoate de glycéryle, le trioctanoate de glycéryle, le tri-2-ethylhexanoate de glycéryle, le triisostéarate de glycéryle, le triisononanoate de glycéryle, le trimyristate de glycéryle,le triisopalmitate de glycéryle, et
- leurs mélanges.

Dans un mode de réalisation, on préfère que l'huile soit un ester choisi parmi les monoesters et les diesters aliphatiques.

Selon un autre mode de mise en oeuvre, l'huile est une huile siliconée comprenant des groupes carbonés aromatiques.

Les huiles polaires préférées de la première composition sont le C12-C15 alkylbenzoate et le néopentanoate d'isodécyle.

### Filmogènes

La première composition est de préférence résistante à l'eau. Dans un mode de mise en oeuvre, elle contient un polymère qui permet de renforcer la cohésivité et l'intégrité et la tenue du dépôt de la composition sur la peau, en particulier au contact de l'eau douce ou salée.

Selon un mode de réalisation, la première composition est sous la forme d'une émulsion contenant de 10 à 20% de filtres UV solaires et au moins un polymère.

On peut citer comme polymères filmogènes, les dérivés de vinyl pyrolidone tels que le VP/HEXADECENE COPOLYMER, le VP/EICOSENE COPOLYMER, et le TRICONTANYL PVP ; les acrylates copolymer ou le Styrène/Acrylates/Ammonium Méthacrylate Copolymer ; les polyuréthanes de type polyurethane-34 ou de type polyurethane-35.

### Emulsion

La première composition contient de l'eau, qui s'évapore au moins partiellement après son application sur la peau. La deuxième composition est avantageusement appliquée sur le dépôt résiduel de la première composition qui a au moins partiellement séché.

Elle est avantageusement sous la forme d'une émulsion eau-dans-huile ou huile-dans-eau, et contient de préférence au moins 20% en poids d'eau, de préférence encore au moins 30% en poids d'eau, et plus préférentiellement de 40 à 80% en poids d'eau par rapport au poids total de la composition. Selon un mode de réalisation, la composition contient de 45 à 50% en poids d'eau. La première composition est sous la forme d'une émulsion contenant au moins un filtre UV liposoluble.

La deuxième composition est appliquée sur la première composition après au moins séchage de celle-ci. Le séchage de la première composition sur la peau correspond à l'évaporation au moins partielle de l'eau qu'elle contient.

L'indice de protection solaire de la première composition diminue au cours du temps, une fois qu'elle a été appliquée sur la peau. La diminution de la protection peut avoir différentes causes selon les cas telles que l'évaporation de l'eau ou des solvants qu'elle contient, la déformation du film de composition formé à la surface de la peau ou la pénétration de certains composés dans la peau. C'est pourquoi, il est préférable d'appliquer la deuxième composition dans un laps de temps tel que l'indice de protection solaire procuré par la première composition n'est pas atteint un seuil trop bas, qui prive le consommateur d'une protection solaire suffisante, efficace et souhaitée.

On préfère par conséquent, dans le cadre de la présente invention, que la deuxième composition soit appliquée sur le dépôt de la première composition dès que l'indice de protection solaire de la première composition atteint un seuil minimum, qui peut être exprimé comme étant supérieur ou égal à 30% de sa valeur initiale, par exemple égal à 35% ; 40% ; 45%, 50%, 55%, 60%, 65%, 70%, 75%, voire même 80% de sa valeur initiale. Dans ce cas, on peut opérer conformément à la procédure décrite dans les exemples qui suivent.

Avantageusement, l'application de la deuxième composition sur le dépôt de la première est effectuée au bout d'une durée d'exposition au soleil n'excédant pas 2 heures, de préférence après un délai d'au moins 30 minutes, d'au moins 45 minutes, d'au moins une heure ou d'au moins une heure 30 minutes, et après l'application de la première composition sur la zone de peau à protéger.

Avantageusement encore, la deuxième composition est appliquée sur la peau, peu de temps après que la zone de peau sur laquelle a été appliquée la première composition a été exposée à de l'eau, par exemple après un bain de mer ou une douche, ou encore après un exercice physique.

La première composition est préférentiellement destinée à être appliquée sur le visage ou le corps et se présente de préférence sous la forme d'une émulsion huile-dans-eau ou eau-dans-huile. La composition est par exemple sous la forme d'une crème, d'une lotion, d'un sérum ou d'un fluide pour le visage ou d'un lait.

### Deuxième composition

La deuxième composition présente un indice de protection solaire SPF inférieur ou égal à 20. De surcroît, la seconde composition cosmétique du kit contient moins de 10% en poids d'eau et au moins une huile volatile. La formule de référence B présente elle-même un SPF de 20.

### Formule de référence B de SPF 20

| **DENOMINATION INCI** | **% Massique** |
|---|---|
| EAU | 63 |
| ETHYLHEXYL METHOXYCINNAMATE | 7,5 |
| CYCLOPENTASILOXANE | 3,1 |
| GLYCEROL | 3 |
| DICAPRYLYL CARBONATE | 3 |
| PENTYLENE GLYCOL | 3 |
| BUTYLENE GLYCOL | 2 |
| BENZOPHENONE-3 | 2 |
| TITANIUM DIOXIDE | 1,8 |
| CETEARYL ALCOHOL | 1,8 |
| GLYCERYL STEARATE | 1,5 |
| PEG-100 STEARATE | 1,2 |
| OCTOCRYLENE | 1 |
| CETYL ALCOHOL | 1 |
| STEARYL ALCOHOL | 1 |
| BETAINE | 1 |
| PHENOXYETHANOL | 0,5 |
| BENZOPHENONE-4 | 0,5 |
| DIMETHICONE | 0,5 |
| CETETH-10 PHOSPHATE | 0,4 |
| DICETYL PHOSPHATE | 0,4 |
| ACIDE STEARIQUE | 0,3 |
| ACRYLATES/C10-30 ALKYL ACRYLATE CROSSPOLYMER | 0,3 |
| HYDROXYDE DE SODIUM | 0,2 |

La deuxième composition présente un indice de protection solaire SPF, inférieur ou égal à 20, de préférence inférieur ou égal à 10.

La deuxième composition présente avantageusement un indice de protection solaire SPF, supérieur ou égal à 3, de préférence supérieur ou égal à 5, de préférence encore supérieur ou égal à 6.

Selon un mode de réalisation, la deuxième composition contient un filtre UV organique et une huile volatile, et son SPF est compris entre 5 et 20, de préférence entre 7 et 10.

### Filtres UV

La deuxième composition contient au moins un filtre UV qui peut être choisi parmi les filtres UV organiques hydrophiles, les filtres UV organiques liposolubles, les filtres UV minéraux, et leurs mélanges.

La deuxième composition contient de préférence au moins un filtre UV organique. Elle est de préférence exempte de filtres UV minéraux responsables d'une texture plus lourde au toucher.

La deuxième composition contient de préférence au moins un filtre UV organique liposoluble tel que défini précédemment.

Chaque composition contient de préférence au moins un filtre UV filtrant les UV_{A} et au moins un filtre UV filtrant les UV_{B}.

La teneur en filtre UV dans la deuxième composition est par exemple comprise entre 2 à 20 % en poids, particulièrement de 4 à 15 % en poids, plus particulièrement de 10 à 15 % en poids, par rapport au poids total de la composition.

La deuxième composition comprend de préférence les filtres UV suivants, pris seuls ou en mélange : le Diéthylhexyl Butamido Triazone, le Butyl Méthoxydibenzoylméthane, l'Octocrylène, la Benzophénone-3, l'Ethylhéxyl Méthoxycinnamate.

### Huile volatile

La deuxième composition contient au moins une huile volatile.

L'huile volatile peut être choisie parmi les huiles hydrocarbonées ayant de 8 à 16 atomes de carbones, et notamment
- les alcanes ramifiés en C8-C16 comme les isoalcanes en C8-C16. notamment l'isododécane (encore appelé 2,2,4,4,6-pentaméthylheptane), l'isodécane, l'isohexadécane,
- les esters ramifiés en C8-C16, comme le néopentanoate d'iso-hexyle,
- les alcools aliphatiques en C2- C5, de préférence l'éthanol.

L'huile volatile peut aussi être choisie parmi les huiles de silicones linéaires ou cycliques, notamment celles ayant une viscosité inférieure à 6 centistokes, et ayant notamment de 3 à 6 atomes de silicium, ces silicones comportant éventuellement un ou plusieurs groupes alkyles ou alkoxy ayant de 1 ou 2 atomes de carbone.

Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane, le 3-butyl 1,1,1,3,5,5,5-heptaméthyl trisiloxane, le 3-propyl 1,1,1,3,5,5,5-heptaméthyl trisiloxane, le 3-éthyl 1,1,1,3,5,5,5-heptaméthyl trisiloxane et leurs mélanges.

On peut également utiliser comme huile volatile des solvants organiques fluorés.

L'huile volatile est de préférence choisie parmi l'isododécane, le décaméthyl cyclopentasiloxane (D5), l'alcool et leurs mélanges.

L'huile volatile peut représenter de 20 à 80 %, de préférence de 50 à 70 % en poids par rapport au poids total de la composition.

La deuxième composition contient moins de 10% en poids d'eau, de préférence encore moins de 5% en poids d'eau par rapport au poids total de ladite composition.

Selon un mode de réalisation, elle contient une forte proportion d'éthanol, notamment plus de 30%, plus de 40% voire même plus de 50% en poids par rapport au poids de la première composition, de manière à procurer une sensation de fraîcheur au moment de son application sur le dépôt de la première composition.

Selon un mode de réalisation, la deuxième composition contient au moins une huile volatile, est exempte de filtre UV minéral et contient moins de 10% en poids d'eau.

### Spray

La deuxième composition est préférentiellement destinée à être appliquée sur le visage ou le corps et se présente de préférence sous la forme d'une composition comprenant moins de 5% en poids d'eau. La composition est par exemple sous la forme d'une huile, en particulier d'une huile sèche. La deuxième composition peut être avantageusement vaporisée sur la peau sous forme de fines particules au moyen de dispositifs adaptés à la pulvérisation de ce type de composition. Ces dispositifs bien connus de l'homme de l'art comprennent les pompes non-aérosols ou "atomiseurs", les récipients aérosols comprenant un gaz propulseur. Ces derniers sont décrits par exemple dans les brevets US 4,077,441 et US 4,850,517. Les compositions conditionnées en aérosol contiennent en général des agents propulseurs conventionnels tels que par exemple de l'air comprimé, des composés hydrofluorés du dichlorodifluorométhane, du difluoroéthane, du diméthyléther, de l'isobutane, du n-butane, du propane, ou du trichlorofluorométhane.

### Kit

L'invention a pour objet un kit de protection solaire comprenant une première composition de protection contre les rayons UV contenant au moins un filtre UV et ayant un indice de protection solaire SPF supérieur ou égal à 30, et au moins une seconde composition cosmétique de protection contre les rayons UV contenant au moins un filtre UV et ayant un indice de protection solaire SPF inférieur ou égal à 20, les deux compositions étant avantageusement conditionnées de façon séparée, de préférence dans un même emballage.

On entend par «kit» un ensemble d'au moins deux produits cosmétiques présentant des conditionnements et des galéniques différentes destinés à être appliqués sur la peau de façon décalée dans le temps pour procurer une protection vis-à-vis des rayons UV.

La première composition se présente sous la forme d'une émulsion contenant au moins un filtre UV liposoluble. La seconde compostion contient moins de 10 % en poids d'eau et au moins une huile volatile. Dans le kit de l'invention, la première composition est de préférence sous la forme d'une émulsion eau-dans-huile ou huile-dans-eau, et la deuxième composition est de préférence anhydre. On entend par anhydre une composition qui contient moins de 5% en poids d'eau.

Selon un mode de mise en oeuvre du kit de l'invention, la première composition présente un indice de protection solaire SPF, supérieur ou égal à 30, de préférence supérieur ou égal à 40, de préférence encore supérieur ou égal à 50, et la seconde composition présente un indice de protection solaire SPF, inférieur ou égal à 30, de préférence inférieur ou égal à 20 et encore plus préférentiellement encore inférieur ou égal à 10.

Avantageusement, le SPF de chacune des compositions du kit est mentionné sur leur conditionnement.

Le kit de protection solaire comprend par exemple une première composition sous forme d'une crème conditionnée dans un tube, tandis que la deuxième est une huile sèche conditionnée en spray.

Les caractéristiques des première et deuxième compositions décrites en relation avec la méthode de l'invention s'appliquent au kit de l'invention.

Avantageusement, le kit selon l'invention contient un descriptif écrit de la méthode de protection solaire selon l'invention, sur l'emballage ou bien sur un support écrit placé à l'intérieur de l'emballage.

### Utilisation

L'invention a pour objet une deuxième composition cosmétique de protection contre les rayons UV pour son utilisation pour prolonger l'efficacité dans le temps d'une première composition de protection de la peau contre les rayons UV ayant un indice de protection solaire supérieur à de la deuxième composition (voir revendication 10). L'utilisation d'une deuxième protection solaire de la peau inférieur à celui de la formule de référence B prolonge l'efficacité dans le temps d'une première composition de protection de la peau contre les rayons UV ayant un indice de protection solaire supérieur ou égal à celui d'une formule de référence A. L'utilisation d'une deuxième composition cosmétique de protection contre les rayons UV ayant un indice de protection solaire, par exemple un facteur de protection solaire SPF, inférieur à 20 prolonge l'efficacité dans le temps d'une première composition de protection de la peau contre les rayons UV ayant un indice de protection solaire, par exemple un facteur de protection solaire SPF, supérieur ou égal à 30.

Les propriétés sensorielles de la deuxième composition sont avantageusement meilleures que celles de la première.

En particulier la deuxième composition ne laisse aucune sensation de gras sur la peau, et glisse à l'application. Elle laisse de préférence une grande sensation de fraîcheur sur la peau.

L'invention est illustrée plus en détail par les exemples suivants. Dans les exemples, tous les pourcentages sont donnés en poids, sauf indication contraire, et la température est exprimée en degré Celsius sauf indication contraire, et la pression est la pression atmosphérique, sauf indication contraire.

### EXEMPLE :

Cet exemple relate les résultats d'une étude consistant à évaluer l'impact sur la protection solaire, de l'application d'une crème solaire dont la valeur du facteur de protection solaire SPF est égale à 50, puis d'un spray solaire dont la valeur du facteur de protection solaire SPF est égale à 8, 2 heures après une première application de ladite crème solaire.

L'étude a porté sur 10 sujets.

Les compositions testées sont les suivantes :

### Première composition : Crème de protection solaire de SPF 50

| **NOM INCI** | **% Massique** |
|---|---|
| EAU | 50,1 |
| BUTYLENE GLYCOL DICAPRYLATE / DICAPRATE | 10,6 |
| ETHYLHEXYL METHOXYCINNAMATE | 7,5 |
| METHYLENE BIS-BENZOTRIAZOLYL TETRAMETHYLBUTYLPHENOL | 5 |
| DICAPRYLYL CARBONATE | 4 |
| BUTYLENE GLYCOL | 3,7 |
| BIS-ETHYLHEXYLOXYPHENOL METHOXYPHENYL TRIAZINE | 3 |
| DIMETHICONE | 2 |
| CAPRYLYL METHICONE | 2 |
| VP/EICOSENE COPOLYMER | 2 |
| BEHENYL ALCOHOL | 2 |
| GLYCEROL | 2 |
| POTASSIUM CETYL PHOSPHATE | 2 |
| CETEARYL ALCOHOL | 1,2 |
| PHENYLBENZIMIDAZOLE SULFONIC ACID | 1 |
| TROMETHAMINE | 1 |
| PHENOXYETHANOL | 0,9 |

La CREME ci-dessus présente des propriétés sensorielles dégradées par la présence des filtres nécessaires à l'obtention d'un SPF d'une valeur égale à 50.

Deuxième composition : Spray d'huile de protection solaire de SPF 8

| **NOM INCI** | **% Massique** |
|---|---|
| ETHANOL | qsp 100 |
| C12-15 ALKYL BENZOATE | 20,0 |
| CYCLOPENTASILOXANE | 8,0 |
| DIETHYLHEXYL BUTAMIDO TRIAZONE | 5,0 |
| EAU | 3,4 |
| BUTYL METHOXYDIBENZOYLMETHANE | 3,0 |
| OCTOCRYLENE | 2,0 |
| BENZOPHENONE-3 | 1,0 |
| ETHYLHEXYL METHOXYCINNAMATE | 1,0 |
| PARFUM | 0,8 |

La composition huile de type SPRAY est très facile et agréable à appliquer sur la peau. Cependant elle a un facteur de protection solaire SPF faible qui ne permet pas d'assurer une protection suffisante pour une exposition au soleil prolongée et intense.

### Protocole de mesure d'indices de protection solaire, dont le SPF

Le détail de la méthode de mesure du facteur de protection solaire SPF de la composition des produits appliqués sont donnés ci-après.

Le protocole et les conditions de mesure du SPF sont celles données dans la méthode « International Sun Protection Factor Test Method - 2006 » publiée par le Colipa.

On a également mesuré un indice de protection solaire différent du SPF en reprenant le protocole et les conditions de mesure du SPF données dans la méthode « International Sun Protection Factor Test Method - 2006 » publiée par le Colipa, à l'exception de la dose de produit appliquée que l'on a choisie plus proche des conditions réelles d'utilisation par les consommateurs, égale à 0,8 mg/cm².

On procède à deux essais qui diffèrent l'un de l'autre par la quantité de produit appliquée sur la peau.

### 1 - Application à 2 mg/cm².

On mesure la valeur du facteur de protection solaire SPF pour chacune des deux compositions. La mesure est réalisée 20 minutes après application, de façon à laisser sécher la composition appliquée.

| **Compositions testées** | **SPF mesuré** |
|---|---|
| CREME SOLAIRE | 51,8 +/- 6,1 |
| SPRAY SOLAIRE | 9,3 +/- 0,9 |

Les résultats obtenus après mise en oeuvre de la méthode de l'invention sont résumés dans le tableau ci-dessous.

| **Etape de la méthode de l'invention** | **SPF mesuré** |
|---|---|
| 1 - Application de la CREME SOLAIRE | 51,8 +/- 6,1 |
| SPF résiduel, deux heures après application | 34,1 +/- 5,9 |
| 2 - Application du SPRAY SOLAIRE, deux heures après application de la CREME SOLAIRE | 51,6 +/- 6,6 |

### 2 - Application à 0,8 mg/cm².

On mesure la valeur de l'indice de protection solaire selon le protocole décrit précédemment. La mesure d'indice de protection solaire est réalisée 20 minutes après application, de façon à laisser sécher la composition appliquée.

| **Compositions testées** | **Indice mesuré** |
|---|---|
| CREME SOLAIRE | 21,7 +/- 3,4 |
| SPRAY SOLAIRE | 7,8 +/- 1,6 |

Les résultats obtenus après mise en oeuvre de la méthode de l'invention sont résumés dans le tableau ci-dessous.

| **Etape de la méthode de l'invention** | **Indice mesuré** |
|---|---|
| 1 - Application de la CREME SOLAIRE | 21,7 +/- 3,4 |
| SPF résiduel, deux heures après application | 13,2 +/- 2,5 |
| 2 - Application du SPRAY SOLAIRE, deux heures après application de la CREME SOLAIRE | 31 , 3 +/- 5, 2 |

On a constaté la diminution significative de la protection solaire résiduelle de la CREME SOLAIRE, deux heures après application.

Ces études ont permis de montrer que dans les conditions d'application décrites dans la méthode de mesure du SPF publiée par le Colipa (2 mg/cm²), mais aussi dans des conditions d'applications analogues dans lesquelles la quantité de produits appliquée est plus proche des conditions réelles d'application par les utilisateurs (0,8 mg/cm²), la perte de protection de la CREME est compensée après 2h par l'application du SPRAY.

On obtient une protection statistiquement supérieure à la simple addition des deux valeurs de SPF ou des deux indices de protection respectivement.

En effet la somme de la valeur du SPF du dépôt résiduel de la crème au bout de deux heures (34,1) et de la valeur du SPF du dépôt du spray (9,1) est égale à 43.4, tandis que la valeur du SPF résultant de la superposition des deux couches est égale à 51,6 : l'effet est bien synergique.

De même, la somme des indices de protection solaire de chacun des deux dépôts de compositions, est bien inférieure à l'indice de protection solaire des deux dépôts superposés.

Les deux compositions de l'exemple peuvent constituer un kit selon l'invention, vendu avantageusement avec un descriptif de la méthode de protection solaire selon l'invention.

Ainsi le consommateur peut se protéger efficacement et grâce à la présence de la deuxième application, bénéficier d'un confort d'application amélioré.

## Revendications

1. Kit de protection solaire comprenant une première composition de protection contre les rayons UV sous la forme d'une émulsion contenant au moins un filtre UV liposoluble et ayant un indice de protection solaire SPF supérieur ou égal à 30, et au moins une seconde composition cosmétique de protection contre les rayons UV contenant au moins un filtre UV et ayant un indice de protection solaire SPF inférieur ou égal à 20, la seconde composition contenant moins de 10% en poids d'eau et au moins une huile volatile, les deux compositions étant conditionnées de façon séparée dans un même emballage.

2. Kit de protection solaire selon la revendication 1, **caractérisé en ce que** la première composition présente un facteur de protection solaire SPF supérieur ou égal à 40.

3. Kit de protection solaire selon l'une des revendications précédentes, **caractérisé en ce que** la seconde composition présente un indice de protection solaire SPF inférieur ou égal à 10, et supérieur ou égale à 5.

4. Kit de protection solaire selon l'une des revendications précédentes, **caractérisé en ce que** la première composition est une crème en émulsion huile-dans-eau ou eau-dans-huile, tandis que la deuxième est une huile sèche conditionnée en spray contenant de l'éthanol.

5. Kit de protection solaire selon l'une des revendications précédentes destiné à être utilisé dans le traitement de la protection de la peau contre les rayons UV.

6. Kit de protection solaire comprenant deux compositions cosmétiques de protection contre les rayons UV ayant des valeurs d'indice de protection solaire différentes, pour son utilisation pour protéger la peau contre les rayons UV, ladite utilisation consistant :
- à appliquer sur la peau une première composition de protection contre les rayons UV sous la forme d'une émulsion contenant au moins un filtre UV liposoluble et ayant un indice de protection solaire SPF supérieur ou égal à 30,
- à exposer la peau au soleil, puis
- à appliquer sur le dépôt résiduel de la première composition une seconde composition cosmétique contenant au moins un filtre UV et ayant un indice de protection solaire SPF inférieur ou égal à 20, la seconde composition étant exemple de filtre UV minéral et contenant moins de 10% en poids d'eau et au moins une huile volatile.

7. kit selon la revendication précédente, **caractérisée en ce que** la première composition contient de l'eau qui s'évapore au moins partiellement après son application sur la peau, et que la deuxième composition est appliquée sur le dépôt résiduel de la première composition qui a au moins partiellement séché.

8. Kit selon les revendications 6 ou 7, **caractérisée en ce que** l'application de la deuxième composition sur le dépôt de la première est effectuée au bout d'une durée d'exposition au soleil n'excédant pas 2 heures, de préférence après un délai d'au moins 30 minutes, d'au moins 45 minutes, d'au moins une heure ou d'au moins une heure 30 minutes, et après l'application de la première composition sur la zone de peau à protéger.

9. Kit selon la revendication précédente, **caractérisée en ce que** la deuxième composition est appliquée sur la peau peu de temps après un exercice physique ou après que la zone de peau sur laquelle a été appliquée la première composition a été exposée à de l'eau.

10. Utilisation d'une deuxième composition cosmétique de protection contre les rayons UV contenant au moins un filtre UV et ayant un indice SPF inférieur ou égal à 20, ladite seconde composition contenant moins de 10% en poids d'eau et au moins une huile volatile, pour prolonger l'efficacité dans le temps d'une première composition de protection de la peau contre les rayons UV sous la forme d'une émulsion contenant au moins un filtre UV liposoluble et ayant un indice de protection solaire supérieur ou égal à 30.

## Patentansprüche

1. Sonnenschutz-Set, umfassend eine erste Zusammensetzung zum Schutz vor UV-Strahlung in Form einer Emulsion, die mindestens einen fettlöslichen UV-Filter enthält und einen Lichtschutzfaktor LSF von 30 oder mehr aufweist, und mindestens eine zweite kosmetische Zusammensetzung zum Schutz vor UV-Strahlung, die mindestens einen UV-Filter enthält und einen Lichtschutzfaktor LSF von 20 oder weniger aufweist, wobei die zweite Zusammensetzung weniger als 10 Gew.-% Wasser und mindestens ein flüchtiges Öl enthält und wobei die beiden Zusammensetzungen getrennt in einer gemeinsamen Verpackung enthalten sind.

2. Sonnenschutz-Set gemäß Anspruch 1, **dadurch gekennzeichnet, dass** die erste Zusammensetzung einen Lichtschutzfaktor LSF von 40 oder mehr aufweist.

3. Sonnenschutz-Set gemäß einem der vorangehenden Ansprüche, **dadurch gekennzeichnet, dass** die zweite Zusammensetzung einen Lichtschutzfaktor LSF von höchstens 10 und mindestens 5 aufweist.

4. Sonnenschutz-Set gemäß einem der vorangehenden Ansprüche, **dadurch gekennzeichnet, dass** die erste Zusammensetzung eine Öl-in-Wasser- oder Wasser-in-ÖI-Emulsionscreme ist, während die zweite Zusammensetzung ein als Spray verpacktes trockenes Öl ist, das Ethanol enthält.

5. Sonnenschutz-Set nach einem der vorangehenden Ansprüche, das dazu bestimmt ist, bei der Hautschutzbehandlung gegen UV-Strahlen eingesetzt zu werden.

6. Sonnenschutz-Set, umfassend zwei kosmetische Zusammensetzungen zum Schutz vor UV-Strahlen, die unterschiedliche Werte für den Lichtschutzfaktor aufweisen, zur Anwendung zum Schutz der Haut vor UV-Strahlen, wobei die Anwendung umfasst:
- Auftragen einer ersten Zusammensetzung zum Schutz vor UV-Strahlung in Form einer Emulsion, die mindestens einen fettlöslichen UV-Filter enthält und einen Lichtschutzfaktor LSF von 30 oder mehr aufweist, auf die Haut,
- die Haut der Sonne aussetzen, dann
- Auftragen einer zweiten kosmetischen Zusammensetzung, die mindestens einen UV-Filter enthält und einen Lichtschutzfaktor LSF von 20 oder weniger aufweist, auf die Restschicht der ersten Zusammensetzung, wobei die zweite Zusammensetzung ein Beispiel für einen mineralischen UV-Filter darstellt und weniger als 10 Gew.-% Wasser und mindestens ein flüchtiges Öl enthält.

7. Set gemäß dem vorangehenden Anspruch, **dadurch gekennzeichnet, dass** die erste Zusammensetzung Wasser enthält, das nach dem Auftragen auf die Haut zumindest teilweise verdunstet, und dass die zweite Zusammensetzung auf die Restschicht der ersten Zusammensetzung, die zumindest teilweise getrocknet ist, aufgetragen wird.

8. Set gemäß den Ansprüchen 6 oder 7, **dadurch gekennzeichnet, dass** die Anwendung der zweiten Zusammensetzung auf die erste Schicht nach einer Dauer der Sonnenexposition von nicht mehr als 2 Stunden, vorzugsweise nach einer Zeitspanne von mindestens 30 Minuten, mindestens 45 Minuten, mindestens einer Stunde oder mindestens einer Stunde 30 Minuten, und nach der Anwendung der ersten Zusammensetzung auf dem zu schützenden Hautbereich durchgeführt wird.

9. Set gemäß dem vorangehenden Anspruch, **dadurch gekennzeichnet, dass** die zweite Zusammensetzung kurz nach einer körperlichen Betätigung oder nachdem der Hautbereich, auf den die erste Zusammensetzung aufgetragen wurde, Wasser ausgesetzt wurde, auf die Haut aufgetragen wird.

10. Anwendung einer zweiten kosmetischen Zusammensetzung zum Schutz vor UV-Strahlung, die mindestens einen UV-Filter enthält und einen LSF von 20 oder weniger aufweist, wobei die zweite Zusammensetzung weniger als 10 Gew.-% Wasser und mindestens ein flüchtiges Öl enthält, zur Verlängerung der Wirksamkeit einer ersten Zusammensetzung zum Schutz der Haut gegen UV-Strahlung in Form einer Emulsion, die mindestens einen fettlöslichen UV-Filter enthält und einen Lichtschutzfaktor von 30 oder mehr aufweist.

## Claims

1. A sun protection kit comprising a first composition for protecting against UV rays in the form of an emulsion containing at least one liposoluble UV-screening agent and having a sun protection index SPF of more than or equal to 30, and at least a second cosmetic composition for protecting against UV rays containing at least one UV-screening agent and having a sun protection index SPF of less than or equal to 20, the second composition containing less than 10% by weight of water and at least one volatile oil, and the two compositions being packaged separately in one packaging.

2. The sun protection kit as claimed in claim 1, **characterized in that** the first composition has a sun protection factor SPF of more than or equal to 40.

3. The sun protection kit as claimed in one of the preceding claims **characterized in that** the second composition has a sun protection index SPF less than or equal to 10, and more than or equal to 5.

4. The sun protection kit as claimed in one of the preceding claims **characterized in that** the first composition is a cream made of oil-in-water or water-in-oil emulsion, whereas the second composition is a dry oil packaged as a spray containing ethanol.

5. The sun protection kit as claimed in one of the preceding claims for its use in a method for protecting the skin against UV rays.

6. A sun protection kit comprising two cosmetic compositions for protecting against UV-rays which have different sun protection index values, for its use in a method for protecting the skin against UV rays, said use consisting in:
- applying on skin a first composition for protecting against UV rays in the form of an emulsion containing at least one liposoluble UV-screening agent and having a sun protection index SPF of more than or equal to 30,
- exposing the skin to sunlight, and then
- applying onto the residual deposit of the first composition a second cosmetic composition containing at least one UV-screening agent and having a sun protection index SPF of less than or equal to 20, the second composition containing at least 10% by weight of water and at least one volatile oil.

7. The kit as claimed in the preceding claim, **characterized in that** the first composition contains water which at least partially evaporates after its application on skin, and **in that** the second composition is applied onto the residual deposit of the first composition which has at least partially dried.

8. The kit as claimed in claim 6 or 7, **characterized in that** the application of the second composition onto the deposit of the first is performed after a duration of sunlight exposure not exceeding 2 hours, preferably after an interval of at least 30 minutes, of at least 45 minutes, of at least one hour or of at least 1 hour 30 minutes, the duration starting at the application of the first composition onto the region of skin to be protected.

9. The kit as claimed in the preceding claim, **characterized in that** the second composition is applied to the skin shortly after a physical exercise or after that the region of skin, which the first composition has been applied onto, has been exposed to water.

10. The use of a second cosmetic composition for protecting skin against UV rays containing at least one UV-screening agent and having a sun protection index of less than or equal to 20, said second composition containing less than 10% by weight of water and at least one volatile oil, for prolonging the efficacy over time of a first composition for protecting the skin against UV rays in the form of an emulsion containing at least one liposoluble UV-screening agent and having a sun protection index of more than or equal to 30.
